(19) Europäisches Patentamt
European Patent Office
Office européen des brevets

(11) Publication number: **0 348 725 A2**

(12) # EUROPEAN PATENT APPLICATION

(21) Application number: 89110654.4

(22) Date of filing: 13.06.89

(51) Int. Cl.4: C07K 7/08 , A61K 39/118 , G01N 33/569

(30) Priority: 29.06.88 US 212846

(43) Date of publication of application: 03.01.90 Bulletin 90/01

(84) Designated Contracting States: DE FR GB

(71) Applicant: MOLECULAR DIAGNOSTICS, INC.
400 Morgan Lane
West Haven, CT 06516(US)

(72) Inventor: Knowles, William J.
85 Fawnbrook Circle
Madison, CT 06443(US)
Inventor: Marchesi, Vincent T.
179 Prospect Avenue
Guilford, CT 06437(US)
Inventor: Huguenel, Edward
11 Beech Road
Guilford, CT 06437(US)
Inventor: Ohlin, Ann C.
150 Calamus Meadow
Hamden, CT 06514(US)

(74) Representative: Dänner, Klaus, Dr. et al
c/o Bayer AG Konzernverwaltung RP Patente Konzern
D-5090 Leverkusen 1 Bayerwerk(DE)

(54) **Species-specific epitode of chlamydia trachomatis and antibodies that recognize the same.**

(57) An antibody against a common epitope of Chlamydia trachomatis, the antibody produced by immunizing an animal against a peptide, the peptide having at least five amino acids, said peptide consisting essentially of at least four amino acids having a sequence position of I to XII of the following epitope-specific natural sequence:

```
Thr-Val-Phe-Asp-Val-Thr-Thr-Leu-Asn-Pro-Thr-Ile
 I   II  III  IV  V   VI  VII VIII IX   X   XI  XII,
```

wherein said at least four amino acids are in their sequence position, but need not be contiguous to one another, the remaining amino acids being in said natural sequence or being immunogenic equivalents to the amino acids in the natural sequence, said peptide having no more than fifteen consecutive amino acids of natural Chlamydia trachomatis antigen and isolating antibody produced thereby.

EP 0 348 725 A2

# Species-specific epitode of chlamydia trachomatis and antibodies that recognize the same

## BACKGROUND OF THE INVENTION

### Field of the Invention

The present invention concerns an antibody against a species-specific epitope of Chlamydia trachomatis and a method of making the same. The invention is also directed to a vaccine for protecting a patient against Chlamydia trachomatis. The present invention also relates to a process for identifying and defining a common epitope for serologically related serotypes of a microorganism, e.g., Chlamydia trachomatis. The invention is also directed to a peptide characterizing a common epitope of Chlamydia trachomatis. Still further, the present invention relates to a nucleic acid probe for detecting Chlamydia trachomatis - specific DNA or RNA sequences.

### Background Information

Chlamydia trachomatis, hereinafter referred to as C. trachomatis or Chlamydia is an obligate, intracellular Gram negative bacterium that causes a number of diseases in humans. The species can be subdivided into 15 distinct serotypes (serovars) based on serological similarities and dissimilarities among them. Each of the serotypes displays a preferred body site for infection. As a sexually transmitted disease, C. trachomatis infection can present a number of distinct manifestations (urethritis, cervicitis, for example). The most serious complications occur in women, where cervical infection can spread to the fallopian tubes, leading to scarring and eventually to infertility. Another serious complication of cervical infection occurs in pregnant women, where newborn infants can contract C. trachomatis infections by passage through the infected birth canal. These newborn infections can involve the infant's genitalia, eyes or lungs, with the lung infection showing high rate of mortality if not detected rapidly.

Due to the fact that individuals often show only mild symptoms, or sometimes remain asymptomatic after infection, many individuals are unaware of infection and C. trachomatis can, and has, spread through the population. By current estimates, three to five million individuals in the United States alone become genitally infected with C. trachomatis each year.

Conventional diagnosis of chlamydial infection can be difficult, requiring a highly trained staff and facilities to support tissue culture work. The conventional diagnosis involves incubating a clinical sample with mammalian cells in culture. Tissue culture cells infected by Chlamydia present in the clinical sample will show characteristic Chlamydia-filled inclusions.

The following investigators have concentrated their efforts in the study of the major outer membrane protein (MOMP) of Chlamydia trachomatis: R. S. Stephens, G. Mullenbach, R. Sanchez-Pescador and N. Agabian, Sequence Analysis of the Major Outer Membrane Protein Gene from Chlamydia trachomatis Serovar $L_2$", Journal of Bacteriology, Vol. 168, No. 3, (1986); R. S Stephens, C-C. Kuo, G. Newport and N. Agabian, "Molecular Cloning and Expression of Chlamydia trachomatis Major Outer Membrane Protein Antigens in Escherichia coli, Infection and Immunity, Vol. 47, No. 3, 713-718, (1985); R. S. Stephens, M. R. Tam, C-C. Kuo, and R. C. Nowinski, "Monoclonal Antibodies to Chlamydia trachomatis: Antibody Specificities and Antigen Characterization", The Journal of Immunology, Vol. 128, No. 3, 1083-1089, 1982); R. S. Stephens, C. J. Inouye and E. A. Wagar, "A Species-Specific Major Outer Membrane Protein Domain", Chlamydial Infections, D. Oriel et al eds., Cambridge University Press, 110-113, (1986); R. S. Stephens, R. Sanchez-Pescador, E. A. Wagar, C. Inouye and M. S. Urdea, "Diversity of Chlamydia trachomatis Major Outer Membrane Protein Genes," J. Bact., 169, 3879-3885, (1987); and R. S. Stephens, E. A. Wagar and G. K. Schoolnik, "High Resolution Mapping of Serovar-Specific and Common Antigenic Determinants of the Major Outer Membrane Protein of Chlamydia trachomatis", J. Exp. Med., 167, 817-831, (1988).

U.S.P. 4,427,782 to Caldwell and Schacter describes a method for isolating the major outer membrane protein and an antigen of Chlamydia trachomatis.

U.S.P. 4,118,469 to Caldwell, Kuo and Kenney describes a Chlamydia trachomatis specific antigen useful for the serological diagnosis of lymphogranoloma venereum.

U.S.P. 4,497,899 to Armstrong, Herrmann and Howard describes a solid phase immunoassay for the detection of Chlamydia trachomatis antigens in a clinical specimen.

| DEFINITIONS | | |
|---|---|---|
| Amino Acid Abbreviations | | Amino Acids |
| D | Asp | aspartic acid |
| N | Asn | asparagine |
| T | Thr | threonine |
| S | Ser | serine |
| E | Glu | glutamic acid |
| Q | Gln | glutamine |
| P | Pro | proline |
| G | Gly | glycine |
| A | Ala | alanine |
| C | Cys | cysteine |
| V | Val | valine |
| M | Met | methionine |
| I | Ile | isoleucine |
| L | Leu | leucine |
| Y | Tyr | tyrosine |
| F | Phe | phenylalanine |
| W | Trp | tryptophan |
| K | Lys | lysine |
| H | His | histidine |
| R | Arg | arginine |

## SUMMARY OF THE INVENTION

It is an object of the invention to provide antibodies against a species-specific epitope of Chlamydia trachomatis.

It is a further object of the present invention to provide a species-specific epitope for Chlamydia trachomatis.

It is another object of this invention to provide a composition for treating a patient to passively protect the patient against infection by Chlamydia trachomatis.

It is another object of the present invention to provide a process for identifying and defining a species-specific epitope for serologically related serotypes of a microorganism, e.g., Chlamydia trachomatis.

It is a further object of the invention to provide a peptide characterizing a species-specific epitope of Chlamydia trachomatis.

It is still a further object of the present invention to provide a nucleic acid probe for detecting Chlamydia trachomatis - specific DNA or RNA sequences.

The above objects and other objects and advantages are satisfied by the present invention.

The present invention concerns an anti-peptide antibody against a species-specific epitope of Chlamydia trachomatis, said antibody produced by immunizing an animal against a peptide, said peptide having at least five amino acids, said peptide consisting essentially of at least four amino acids having a sequence position of I to XII of the following epitope-specific natural sequence:

| Thr | Val | Phe | Asp | Val | Thr | Thr | Leu | Asn | Pro | Thr | Ile |
|---|---|---|---|---|---|---|---|---|---|---|---|
| I | II | III | IV | V | VI | VII | VIII | IX | X | XI | XII, |

wherein the at least four amino acids are in their sequence position, but need not be contiguous to one another, the remaining amino acids being in said natural sequence or being immunogenic equivalents to the amino acids in the natural sequence, the peptide having no more than fifteen consecutive amino acids of natural Chlamydia trachomatis antigen and isolating antibody produced thereby. More particularly, the

epitope-specific sequence is
Thr-$AA_1$-Phe-$AA_2$-Pro-$AA_3$-Ile
wherein $AA_1$, $AA_2$ and $AA_3$ are, respectively, Val, Asp-Val-Thr-Thr-Leu-Asn and Thr, or immunogenic equivalents thereof, and isolating antibody produced thereby.

The present invention also relates to a peptide (Chlamydia trachomatis eptitope-specific peptide) having at least five amino acids, (preferably having seven or eight amino acids), the peptide consisting essentially of at least four amino acids having a sequence position of I to XII of the following epitope-specific natural sequence:

| | | | | | | | | | | | |
|---|---|---|---|---|---|---|---|---|---|---|---|
| Thr- | Val- | Phe- | Asp- | Val- | Thr- | Thr- | Leu- | Asn- | Pro- | Thr- | Ile |
| I | II | III | IV | V | VI | VII | VIII | IX | X | XI | XII, |

wherein the at least four amino acids are in their sequence position, but need not be contiguous to one another, the remaining amino acids being in the natural sequence or being immunogenic equivalents to the amino acids in the natural sequence, the peptide having no more than fifteen consecutive amino acids of natural Chlamydia trachomatis antigen. More particularly, the epitope-specific sequence of the peptide is Thr-Val-Phe-Asp-Val-Thr-Thr-Leu-Asn-Pro-Thr-Ile.

The present invention is also directed to a vaccine for treating patients, e.g., warm blooded animals, e.g., humans, to protect against infection by Chlamydia trachomatis, i.e., as a protection against contracting Chlamydia trachomatis comprising an effective amount of a peptide as described hereinabove in a physiologically acceptable diluent. The invention also relates to a prophylaxis method for treating patients, e.g., warm blood animals, e.g., humans, infected with Chlamydia trachomatis comprising an effective amount of a peptide as described hereinabove in a physiologically acceptable diluent.

The present invention also relates to a process for identifying and defining a common epitope for serotypes of a microorganism, e.g., Chlamydia trachomatis, comprising

(1) generating antibodies which react with said common epitope, said antibodies generated by

(a) immunizing an animal with a first serotype of the microorganism or a fragment thereof,

(b) boosting the immune response of the animal by immunizing said animal with a second, distantly related serotype of the microorganism,

(c) identifying and then generating monoclonal antibodies and/or polyclonal antibodies that are cross reactive with the different serotypes of the microorganism,

(2) identifying the chemical structure of the common epitope, comprising

(a) fragmenting the microorganism to product proteins,

(b) fragmenting the proteins into smaller portions,

(c) ascertaining which protein fragment contains the common epitope by reacting the protein fragments with the antibodies from step (1)(c) and determining which protein fragments bind to the antibodies,

(d) isolating the protein fragments that bind to the antibodies,

(e) determining the amino acid sequence of the isolated protein fragments from step (2)(d).

The invention is further directed to a process for making antibodies reactive with a common epitope of a microorganism comprising

(a) synthesizing a peptide having the amino acid sequence of step (2)(e) of the above described process,

(b) immunizing an animal against said synthesized amino acid sequence and

(c) isolating antibodies produced thereby.

The present invention is further directed to a nucleic acid probe for detecting Chlamydia trachomatis - specific DNA or RNA sequences, consisting essentially of less than about 45 DNA or RNA bases and including a sequence which codes for the following epitope-specific peptide sequence:
Thr-Val-Phe-Asp-Val-Thr-Thr-Leu-Asn-Pro-Thr-Ile.

The present invention also relates to a method for detecting Chlamydia trachomatis in a test sample, the method comprising contacting the sample with an anti-peptide first antibody, the antibody being against a common epitope of Chlamydia trachomatis as defined above and determining binding of the antibody to an antigen in the sample.

The above method can be practiced wherein binding of the antibody to antigen is determined by contact with a labeled form of an epitope-specific peptide, or by contact with a labeled form of the anti-peptide antibody.

The present invention is also directed to a method for immunopurifying a polyclonal antibody against a common epitope of Chlamydia trachomatis, comprising the steps of:

(a) contacting an antiserum preparation, e.g., fractionated IgG, raised against a Chlamydia trachomatis antigen with an epitope-specific peptide having at least five amino acids, such peptide consisting essentially of at least four amino acids having a sequence position of I to XII of the following epitope-specific natural sequence:

```
Thr-Val-Phe-Asp-Val-Thr-Thr-Leu-Asn-Pro-Thr-Ile
 I   II  III  IV  V  VI  VII VIII IX  X   XI  XII,
```

wherein such at least four amino acids are in their sequence position, but need not be contiguous to one another, the remaining amino acids being in such natural sequence or being immunogenic equivalents to the amino acids in the natural sequence, the peptide having no more than 15 consecutive amino acids of natural Chlamydia trachomatis antigen,

(b) separating antibody that becomes bound to the epitope-specific peptide from antibody an any other materials from the antiserum preparation that does not become so bound,

(c) disassociating antibody that had become bound to the peptide, and

(d) isolating the purified, disassociated antibody.

A preferred embodiment of the aforementioned method for immunopurifying a polyclonal antibody against a common epitope of Chlamydia trachomatis would be where

(a) the antiserum preparation is applied to a column containing the peptide fixed to a solid support,

(b) unbound material is eluted, and

(c) epitope-specific antibody is eluted under conditions which disassociate antibody from the solid-phase peptide.

The present invention is further directed to a method for assessing the proper performance of an assay method for detecting Chlamydia trachomatis in a sample, the method comprising:

(a) performing a Chlamydia trachomatis assay on the sample by contacting the sample with a first antibody to Chlamydia trachomatis and detecting whether an antigen from the sample becomes bound to the first antibody, and

(b) performing a control reaction by contacting the first antibody, under conditions substantially the same as in step (a), with an epitope-specific peptide and detecting whether the peptide becomes bound to the first antibody, the peptide having at least five amino acids and consisting essentially of at least four amino acids having a sequence position of I to XII of the following epitope-specific natural sequence:

```
Thr-Val-Phe-Asp-Val-Thr-Thr-Leu-Asn-Pro-Thr-Ile
 I   II  III  IV  V  VI  VII VIII IX  X   XI  XII,
```

wherein the at least four amino acids are in their sequence position, but need not be contiguous to one another, the remaining amino acids being in the natural sequence or being immunogenic equivalents to the amino acids in the natural sequence.

A preferred embodiment of the aforementioned method for assessing the proper performance of an assay method for detecting Chlamydia trachomatis in a sample is wherein the detection of Chlamydia antigen binding to antibody in step (a) is accomplished by adding a second antibody capable of binding to the Chlamydia antigen, which antibody is labeled with a signal producing moiety, and detecting the signal that becomes associated with the first antibody.

In a further preferred embodiment of the aforementioned method for assessing the proper performance of an assay method for detecting Chlamydia trachomatis in a sample is wherein the peptide is labeled with the same signal producing moiety as the second antibody, and step (b) is completed by detecting the signal that has become associated with the first antibody used in such step (b).

In still a further preferred embodiment of the aforementioned method for assessing the performance of an assay method for detecting Chlamydia trachomatis in a sample is wherein multiples of the peptide are linked together to form a multivalent peptide, and step (b) is completed by adding the labeled second antibody and detecting the signal that has become associated with the first antibody used in such step (b).

## BRIEF DESCRIPTION OF THE DRAWINGS

Fig. 1A is a plot of the elution profile from a reverse phase HPLC column of CNBr-digested outer membrane complex of Chlamydia trachomatis.

Fig. 1B is a plot of the elution profile from a reverse phase HPLC column of the monoclonal-reactive fraction shown in Fig. 1A, which has been rechromatographed using a different solvent system.

Fig. 2a is a graph which depicts ELISA results for $NH_2$-terminal "deletions", i.e., the delineation of the $NH_2$-terminus of the Chlamydia trachomatis species-specific epitope.

Fig. 2b is a graph which depicts the ELISA results for COOH-terminal "deletions", i.e., the delineation of the COOH-terminus of the species-specific Chlamydia trachomatis epitope.

Fig. 3 is a graph depicting the ELISA results of internal aa substitutions for the 12 amino acids peptide according to the invention (the asterisks indicate the point of the substitution).

Fig. 4 compares the relative titers of four species-specific antibody reagents by means of a plot.

Fig. 5 is a plot giving the relative affinity of two antibodies for chlamydial outer membrane complex antigen.

Fig. 6 depicts the results of a Western blot analysis of the reactivity of several anti-Chlamydia antibodies with chlamydial outer membrane complex antigen.

Fig. 7 depicts of an indirect immunofluorescence of Chlamydia infected HeLa cells using four antibody reagents.

## DETAILED DESCRIPTION OF THE INVENTION

It has now been determined that the MOMP antigen of Chlamydia trachomatis contains a region that is common among all known serovars. This epitope-specific peptide region has been determined to consist essentially of the amino-acid sequence:

Thr-$AA_1$-Phe-$AA_2$-Pro-$AA_3$-Ile

wherein each of $AA_1$ through $AA_3$ are the amino acids or amino acid sequences existing in the protein sequence of MOMP, namely, $AA_1$ is Val, $AA_2$ is the sequence of Asp-Val-Thr-Thr-Leu-Asn and $AA_3$ is Thr. For purposes of generating antibodies against such common epitope, however, it has thus far been determined that only the Thr, Phe, Pro and Ile so depicted in the formula above are critical and therefore $AA_1$, $AA_2$ and $AA_3$, independently, may be amino acids that are immunogenically equivalent to the normal MOMP sequence. Such equivalent amino acids or amino acid sequences will be determined empirically and will be characterized by amino acid substitutions that do not substantially alter the specificity of antibodies generated thereagainst for the common epitope. While not altering specificity, it is contemplated that certain substitutions will actually result in the generation of antibodies having superior properties, e.g., higher binding affinities for the common epitope.

While the above formula indicates the minimum size of the peptide used to produce epitope-specific antibodies, the peptide used in immunization can include flanking amino acid sequences which may or may not correspond in whole or in part to the sequence of MOMP. Normally, the total length of the peptide will not exceed about 15 amino acids and more preferably will be less than about 8 units.

The peptides of the present invention can be prepared as follows:

(1) peptides prepared by synthetic means, i.e., synthesis of amino acid,

(2) peptides produced by digestion or cleavage,

(3) peptides expressed by recombinant DNA techniques.

Regarding synthetic peptides according to the invention, chemical synthesis of peptides is described in the following publications: S.B.H. Kent, Biomedical Polymers, eds. E.P. Goldberg and A. Nakajima, (Academic Press, New York), 213-242, (1980); A.R. Mitchell, S.B.H. Kent, M. Engelhard and R.B. Merrifield, J. Org. Chem., 43, 2845-2852, (1978); J.P. Tam, T.-W. Wong, M. Riemen, F.-S. Tjoeng and R.B. Merrifield, Tet. Letters, 4033-4036, (1979); S. Mojsov, A.R. Mitchell and R.B. Merrifield, J. Org. Chem., 45, 555-560, (1980); J.P. Tam, R.D. DiMarchi and R.B. Merrifield, Tet. Letters, 2851-2854, (1981); and S.B.H. Kent, M. Riemen, M. Le Doux and R.B. Merrifield, Proceedings of the IV International Symposium on Methods of Protein Sequence Analysis, (Brookhaven Press, Brookhaven, NY), in press, 1981.

In the Merrifield solid phase procedure, the appropriate sequence of L-amino acids is built up from the carboxyl terminal amino acid to the amino terminal amino acid. Starting with the appropriate carboxyl terminal amino acid attached to a polystyrene (or other appropriate) resin via chemical linkage to a chloromethyl group, benzhydrylamine group, or other reactive group of the resin, amino acids are added

one by one using the following procedure. The peptide-resin is:

(a) washed with methylene chloride;

(b) neutralized by mixing for 10 minutes at room temperature with 5% (v/v) diisopropylethylamine (or other hindered base) in methylene chloride;

(c) washed with methylene chloride;

(d) an amount of amino acid equal to six times the molar amount of the growing peptide chain is activated by combining it with one-half as many moles of a carbodiimide (e.g., dicyclohexylcarbodiimide, or diisopropylcarbodiimide) for ten minutes at 0°C, to form the symmetric anhydride of the amino acid. The amino acid used should be provided originally as the N-alpha-tert.butyloxycarbonyl derivative, with side chains protected with benzyl esters (e.g. aspartic or glutamic acids), benzyl ethers (e.g.,serine, threonine, cysteine or tyrosine), benzyloxycarbonyl groups (e.g., lysine) or other protecting groups commonly used in peptide synthesis;

(e) the activated amino acid is reacted with the peptide-resin for two hours at room temperature, resulting in addition of the new amino acid to the end of the growing peptide chain;

(f) the peptide-resin is washed with methylene chloride;

(g) the N-alpha-(tert. butyloxycarbonyl) group is removed from the most recently added amino acid by reacting with 30 to 65%, preferably 50% (v/v) trifluoroacetic acid in methylene chloride for 10 to 30 minutes at room temperature;

(h) the peptide-resin is washed with methylene chloride;

(i) steps (a) through (h) are repeated until the required peptide sequence has been constructed. The peptide is then removed from the resin and simultaneously the side-chain protecting groups are removed, by reaction with anhydrous hydrofluoric acid containing 10% v/v of anisole or other suitable (aromatic) scavenger. Subsequently, the peptide can be purified by gel filtration, ion exchange, high pressure liquid chromatography, or other suitable means.

In some cases, chemical synthesis can be carried out without the solid phase resin, in which case the synthetic reactions are performed entirely in solution. The reactions are similar and well known in the art, and the final product is essentially identical.

Digestion of peptides can be accomplished by using proteolytic enzymes, especially those enzymes whose substrate specificity results in cleavage of the polypeptide at sites immediately adjacent to the desired sequence of amino acids.

Cleavage of a peptide can be accomplished by chemical means. Particular bonds between amino acids can be cleaved by reaction with specific reagents. Examples include the following: bonds involving methionine are cleaved by cyanogen bromide; asparaginyl-glycine bonds are cleaved by hydroxylamine.

It should also be possible to clone a small portion of the DNA, either from natural sources or prepared by synthetic procedures, or by methods involving a combination thereof, that codes for the desired sequence of amino acids, resulting in the production of the peptide by bacteria, or other cells.

If desired the synthetic peptide of the present invention can have bonded thereto a chain of any of the following moieties: polypeptide, polyamino acid, polysaccharide, polyamide or polyacrylamide which can serve as a stabilizing chain or as a bridge between amino acids of the individual chains. Such chains are available commercially or, in the case of polyamino acids, are formed by a process which comprises: mixing a solution of the desired amino acid sequence with a solution of the N-carboxylanhydride of the amino acid and allowing a base-catalyzed polymerization to occur, which is initiated by the amine groups of the peptide.

A suitable animal, e.g., mouse, rabbit, goat, man, etc., can be immunized against the eptiope-specific peptide in any conventional manner. While the peptide is of sufficient size and character, it can serve as the necessary or desirable to link the peptide to an immunogen itself. In many instances however, it will be immunogenic carrier to form an immunogen conjugate.

The "immunogenic carrier" is simply a physiologically acceptable mass to which the synthetic peptide is attached and which is expected to enhance the immune response. A carrier can comprise simply a chain of amino acids or other moieties and to that end it is specifically contemplated to use as a carrier a dimer, oligomer, or higher molecular weight polymer of a sequence of amino acids defining a synthetic peptide of the invention. In other words, having determined the desired sequence of amino acids to form the synthetic peptide, these amino acids can be formed from naturally available materials or synthetically and can be polymerized to build up a chain of two or more repeating units so that repeating sequences serve both as "carrier" and synthetic peptide. Stated differently, an independent carrier may not be required. Alternatively, additional amino acids can be added to one or both ends of the amino acid chain that defines the synthetic peptide. It is preferred that alternative carriers comprise some substance, animal, vegetable or mineral, which is physiologically acceptable and functions to present the synthetic peptide so that it is recognized

by the immune system of a host and stimulates a satisfactory immunological response. Thus, a wide variety of carriers are contemplated, and these include materials which are inert, which have biological activity and/or promote an immunological response. For instance, proteins can be used as carriers. Examples of protein carriers include tetanus toxoid, keyhole lympet hemocyanin, bovine serum albumin, etc.

Polysaccharides are also contemplated as carriers, and these include especially those of molecular weight 10,000 to 1,000,000, including, in particular, starches, dextran, agarose, ficoll or its carboxy methyl derivative and carboxy methyl cellulose.

Polyamino acids are also contemplated for use as carriers, and these polyamino acids include, among others, polylysine, polyalanyl polylysine, polyglutamic acid, polyaspartic acid and poly ($C_2$-$C_{10}$) amino acids.

Organic polymers can be used as carriers, and these polymers include, for example, polymers and copolymers of amines, amides, olefins, vinyls, esters, acetals, polyamides, carbonates and ethers and the like. Generally speaking, the molecular weight of these polymers will vary dramatically. The polymers can have from two repeating units up to several thousand, e.g., two thousand repeating units. Of course, the number of repeating units will be consistent with the use of the vaccine in a host animal. Generally speaking, such polymers will have a lower molecular weight, say between 10,000 and 100,000 (the molecular weight being determined by ultracentrifugation).

Inorganic polymers can also be employed. These inorganic polymers can be inorganic polymers containing organic moieties. In particular, silicates and aluminum hydroxide can be used as carriers. It is preferred that the carrier be one which is an immunological adjuvant. In such cases, it is particularly contemplated that the adjuvant be muramyl dipeptide or its analogs.

The carrier can also be the residue of a crosslinking agent employed to interconnect a plurality of synthetic peptide containing chains. Crosslinking agents which have as their functional group an aldehyde (such as glutaraldehyde), carboxyl, amine, amido, imido or azidophenyl group. In particular, there is contemplated the use of butyraldehyde as a crosslinking agent, a divalent imido ester or a carbodiimide. Particularly contemplated divalent imido esters are those of the formula

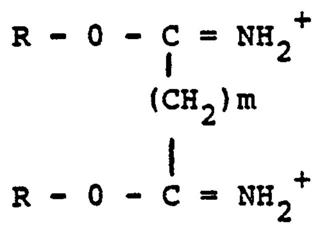

wherein m is 1 to 13 and R is an alkyl group of 1 to 4 carbon atoms. Particularly contemplated carbodiimides for use as crosslinking agents include cyclohexylcarboxiimide, ethyldimethylaminopropyl carbodiimide, N-ethylmorpholino cyclohexyl carbodiimide and diisopropyl carbodiimide.

Although a carrier may not be required, if a carrier is employed the deposition of a chain or chains on a "carrier" can be effected as follows:

1. Protein Carrier: The protein and the synthetic peptide are dissolved together in water or other suitable solvent, and covalently linked via amide bonds formed through the action of a carbodiimide. The resulting product may contain one or more copies of the peptide per protein monomer. Alternatively, the reduced peptide may be added to a carrier containing sulfhydryl groups to form disulfide bonds. Yet another method involves the addition of reduced peptide to protein carriers containing maleimidyl groups to form a covalent linkage by a Michael addition, or any other covalent attachment means.

2. Polysaccharide Carriers: Oligosaccharide carriers should have molecular weights in the range 1,000 to 1,000,000. In order to covalently link these to synthetic peptides, suitable functional groups must first be attached to them. Carboxyl groups may be introduced by reacting with iodoacetic acid to yield carboxymethylated polysaccharides, or by reacting with carbonyldiimidazole to yield activated carbonyl esters. Carboxymethyl polysaccharides are coupled to the peptide by a carbodimide reaction, while the activated carbonyl esters react spontaneously with peptides. Multiple copies of the synthetic peptide should be attached to each oligosaccharide unit.

3. Polyamino Acid Carriers: These carriers should have molecular weights in the range 1,000 to 1,000,000. Polylysine and polyornithine have primary amino groups on their side chains; polyaspartic acid and polyglutamic acid have carboxyl groups. Peptides may be coupled to these via amide bonds using the carbodiimide reaction. Another carrier that provides amino groups for coupling is polylysine to which

polyalanine can be attached to the side chains of the lysine residues. The synthetic peptide may be attached to the ends of polyalanine chains, also by a carbodiimide reaction. Multiple copies of the synthetic peptide should be attached to each oligopeptide unit.

There is realized by the present invention a synthetic vaccine which is characterized by the absence of the naturally occurring MOMP protein Chlamydia trachomatis.

The peptide of the present invention is such that it is capable of forming "neutralizing antibodies", i.e., antibodies that will protect patients against Chlamydia trachomatis. Accordingly, the present invention is also directed to methods for protecting a patient against contracting Chlamydia trachomatis.

The active component of a vaccine according to the invention can be employed with a physiologically acceptable diluent (medium), e.g., phosphate buffered saline.

The vaccine can be administered by subcutaneous, intradermal or intramuscular injection. While the preferred route would depend upon the particular vaccine, it is believed that intramuscular injection will be generally suitable. Frequency of administration will vary depending upon the vaccine. Generally speaking, the vaccine will be administered in two doses about one month apart followed by a booster at six months to one year after primary immunization. The subsequent doses or the booster will depend on the level of antibody in the blood as a result of the initial immunization, and in certain instances may be unnecessary.

The peptide of the present invention can also be used in a prophylaxis method to block the binding of the organism to receptors on the surface of the host cells.

To generate monoclonal antibodies according to the present invention, animals, e.g., mice are immunized with the aforementioned peptide, preferably with a carrier, e.g., KLH or BSA. Monoclonal antibodies are then generated by the G. Kohler and C. Milstein approach (European Journal of Immunology, Vol. 6, pages 511-519, (1976)). More specifically, lymphocytes such as spleen cells from the immunized mice are fused with myeloma cells, e.g., mice myeloma cells, to form hybrid-myeloma clones (hybridomas). Each of the hybridomas secrete monoclonal antibodies. The hybridomas, which are preferably formed by a "mouse-mouse" fusion, i.e., mouse (murine) spleen cells and mouse (murine) myeloma cells, are cultured and screened for production of anti-Chlamydia trachomatis antibodies. Screening can be accomplished using a standard microimmunofluorescence assay.

The present invention is directed to a method for detecting Chlamydia trachomatis in a test sample, comprising the steps of contacting the sample with an anti-peptide antibody as described above and determining binding of the anti-peptide antibody to an antigen in the sample.

In the above method, the binding of the antibody to antigen in the sample can be determined by contact with a labeled, second antibody capable of binding to said antigen.

In the above method, the anti-peptide antibody and the second antibodies, independently of each other, can be polyclonal or monoclonal antibodies.

In the above method, the anti-peptide antibody can be immobilized on a solid support.

In the above method, the binding of the anti-peptide antibody to antigen in the sample can be determined by contact with a labeled form of an epitope-specific peptide as described hereinabove or by using a labeled form of the anti-peptide antibody.

The invention also concerns kits for carrying out the above described method.

One kit comprises one or more containers of the following:

(a) an anti-peptide first antibody against a common epitope of Chlamydia trachomatis as defined above, and

(b) a labeled second antibody, the second antibody capable of binding to Chlamydia trachomatis antigen.

Another kit comprises one or more containers of the following:

(a) an anti-peptide antibody against a common epitope of Chlamydia trachomatis as defined above, and

(b) a labeled form of an epitope-specific peptide of Chlamydia trachomatis.

In the above methods and kits, the label may be a radiolabel, an enzyme, a dye, a fluorescent moiety, biotin-avidin, or any other label used in the art.

In the above methods and kits, the antibody against a common epitope of Chlamydia trachomatis may be immobilized on a solid support.

The invention will now be described with reference to the following non-limiting examples.

## EXAMPLES

Example 1: Chlamydia Cultivation and Purification

Chlamydia trachomatis $L_2$/434/Bu and C/tw-3/ot isolates were cultivated in HeLa 229 cells, according to the method of C.-C. Kuo, S.-P. Wong and J. T. Grayston, "Growth of Trachoma Organisms in Hela 229 Cell Culture", Nongonococcal Urethritis and Related Infections, D. Hobson and K. K. Holmes, eds., American Society for Microbcology, Washington, D.C. 328-336. Briefly, C. trachomatis concentrated inoculum was diluted in SPG buffer (0.2 M sucrose, 3 mM $K_2HPO_4$, 8.5 mM $Na_2HPO_4$, 5.6 mM L-glutamic acid) and 2.5mL of the diluted inoculum was applied to each 150 $cm^2$ tissue culture flask, containing a confluent HeLa 229 monolayer, from which the tissue culture medium had been removed prior to addition of the inoculum. In the case of the C serotype, before addition of the inoculum, the HeLa monolayer was pretreated with 10 mL of Hanks Balanced Salt Solution (HBSS), pH 7.0 containing 30µg/ml DEAE-dextran (Pharmacia, Upsala, Sweden) for 30 minutes at 37° C. The dextran solution was removed, the monolayer washed once with 10 mL HBSS and then the inoculum was applied as described above. Each inoculated flask was placed on a rocker platform and rocked for two hours at room temperature. The inoculum was then removed from each flask by aspiration and replaced with 50 mL Dulbecco's Modified Eagle's Medium (DMEM; Hazelton, Denver, Pa., U.S.A.) containing 10% fetal calf serum, 2mM L-glutamine, 0.2 ng/ml vancomycin and 100µg/mL streptomycin. The flasks were incubated at 37° C in a 5% $CO_2$ atmosphere. After 16 hours of incubation, emetine was added to the culture medium to a concentration of 1 µg/mL. The infected cultures were then examined periodically over the next 24-36 hours for the appearance of elementary bodies (EBs) within cellular inclusions. Typically, production of EBs would peak at approximately 42 hours after infection, at which time the EBs would be harvested. Harvest of EBs was accomplished by first removing the medium from the tissue culture flasks and replacing it with 10mL of ice cold PBS (50 mM $NaPO_4$, 150 mM NaCl, pH 7.4). Adherent HeLa cells were removed from the surface of the flask by adding sterile glass beads to each flask and tilting the flask back and forth so that the beads rolled over the monolayer, removing it from the surface of the flask. The PBS was then removed and the procedure repeated with a fresh 10 mL aliquot of PBS. The pooled cell suspension was then sonicated for a total of 30 individual one second bursts at 50% power level (Branson Cell Disruptor 200 Sonifier, Microtip sonicator probe). The sonicated suspension was centrifuged at 1000xG for 10 minutes to remove cellular debris. The supernatant was recentrifuged at 15,000xG for 60 minutes to pellet EBs. The pellets were resuspended in HBSS and applied to Renografin step gradients as described by H. D. Caldwell, J. Kromhout and J. Schachter, "Purification and Partial Characterization of the Major outer Membrane Protein of Chlamydia trachomatis", Infection and. Immunity, 31, 1161-1176, (1981). The gradients were centrifuged at 18,000 RPM for 60 minutes in an SW-28 (Beckman, Carlsbad, (Cal., U.S.A.) swinging bucket rotor. The EBs, which band at the 44%-54% Renografin interface, were removed, diluted 3X in HBSS and centrifuged at 20,000xG for 35 minutes. The pellet, containing purified EBs, was resuspended in SPG for storage by freezing.

Example 2: Preparation of Chlamydia Outer Membrane Complexes

Purified EBs ($10^{10}$-$10^{11}$) were removed from SPG storage medium by centrifugation and the pellet was extracted in 5 ml PBS (50mM sodium phosphate, pH 7.4, 0.15M NaCl) containing 1% sodium lauroylsarcosine ("Sarkosyl") for one hour at 37° C. The extract was ten centrifuged at 100,000g for 45 minutes at 20° C. The Sarkosyl-insoluble pellet, containing outer membrane complexes ("COMCs"; Caldwell, Kromhout and Schachter, supra.) was suspended in PBS.

Example 3: Hybridoma Preparation

Six to eight week old Balb C/BYJ mice (Jackson Labs, Bar Harbor, Me., U.S.A.) were each immunized subcutaneously with 33 µg of serotype $L_2$ COMCs in 50% (v/v) Freund's complete adjuvant. Four days before fusion, mice were each boosted intravenously with 20 µg of ethanol-fixed serotype C EBs in PBS and intraperitoneally with 10 ug of serotype C EBs. In some experiments, each mouse was given an additional immunization, administered intraperitoneally 30 days after the primary, of 20 µg serotype $L_2$ COMC in PBS and 50% (v/v) Freund's incomplete adjuvant.

Hybridomas were generated through fusion of immunized mouse spleen cells with P3-X63AG8 cells (ATCC No. CRL 1580) (American Type Culture Collection) by the method of G. Kohler and C. Milstein", Continuous Cultures of Fused Cells Secreting Antibody of Predefined Specificity", Nature, 256, 495-497, (1975) and cultivated in RPMI 1640 supplemented with 20% fetal calf serum, 2mM L-glutamine, 100

units/ml penicillin, 100 µg/mL streptomycin and 1% HAT ($10^{-2}$ mM sodium hypoxanthine, $40^{-2}$ µM aminopterin and $1.6^{-2}$ mM thymidine).

Example 4: Identification of anti-Chlamydia Hybridomas

Hybridoma supernatants were assayed by an enzyme-linked immunosorbent assay (ELISA; E. Engvall and P. Perlmann, "Enzyme Linked Immuno Sorbent Assay (ELISA). Quantitative Assay of Immunoglobulum G.", Immuno Chem., 8 821, (1971)) method to detect anti-Chlamydia antibodies. The ELISA antigen was prepared by adjusting a COMC suspension (see Example 2) to 1% sodium dodecyl sulfate (SDS) and 10 mM dithiothreitol (DTT) and incubating for 30 minutes at 37° C. The solublized COMCs were then alkylated by adding 1/10 vol of 500 mM iodoacetimide, and incubated for 30 minutes at room temperature. The alkylated material was diluted in PBS, pH 7.4 containing 0.1% SDS and 1 mM DTT to a final protein concentration (O. H. Lowry, N. J. Rosebrough, A. L. Farr and R. J. Randall, "Protein Measurement With the Folin Reagent", J. Biol. Chem., 193, 265-275, (1951)) of 75-100 µg/ml and this stock ELISA antigen was stored in 100 µl aliquots in siliconized tubes at -80° C. The day before hybridoma screening, stock ELISA antigen was diluted 1:100 in PBS, pH 7.4 and applied to microtiter plates (Immulon I, Dynatech) for coating at 100 µl/well. Plates were incubated 16-20 hours at 4° C. The coating solution was removed, and plates were blocked with 200 µl/well PBST (PBS, pH 7.4 containing 0.05% "TWEEN-20") plus 1% BSA (bovine serum albumin, Sigma Fraction V RIA grade) for 45 minutes at room temperature. The blocking solution was removed, and 100 µl of the culture medium from hybridomas was added to the wells. The plates were incubated for one hour at room temperature to allow anti-Chlamydia antibody to bind to the immobilized COMC antigen. Sterile hybridoma culture medium was used as a negative control, and serum from the immunized mice used in the fusion process served as a positive control. The plates were then washed five time with 200 µl/well PBST and each well was incubated for 60 minutes with 100 µl of peroxidase-conjugated goat anti-(mouse IgG) (heavy and light chain specific, Cappel Laboratories, Malvern, Pa., U.S.A.) antibody at a 1:500 dilution in PBST containing 1% BSA. Plates were washed six times as described above and bound antibody detected by incubating each well with 150 µl of enzyme substrate (40 mg o-phenylenediamine (Kodak, Rochester, New York, U.S.A.) 40 µl 30% hydrogen peroxide (Mallinkrodt, St. Louis, Mo., U.S.A.), in 100 ml 25 mM citric acid, 50 mM sodium phosphate, pH 5.0). Plates were incubated with substrate at room temperature on a mini-orbital shaker (Bellco, Vineland, N.J., U.S.A.) for 15 minutes and the reaction stopped by the addition of 50 µl 8 M $H_2SO_4$ to each well. Absorbance of each well at 492 nm was determined using a Titertek (Flow Laboratories, McLean, Va., U.S.A.) spectrophotometer. Hybridomas giving positive ELISA results were cloned by limiting dilution and clones re-tested for reactivity with COMC antigen by ELISA as described above. Positive clones were then grown as ascites, which were propagated in Balb C/BYJ mice primed by intraperitoneal injection of 0.5mL Pristane (Aldrich, Milwaukee, Wisconsin, U.S.A.) 10 days before intraperitoneal injection with $10^7$ hybridoma per cell animal. Ascites and purified IgG (Example 5) were tested in a microimmunofluorescence assay. A number of monoclonal antibodies were identified that reacted with all 15 C. trachomatis serotypes. One of these antibodies, hereinafter referred to as species-specific monoclonal antibody ("SSMAB-1" or "SSM-1") was selected to identify species-specific antigens in experiments described below.

SSMAB-1 is a C. trachomatis species-specific monoclonal antibody which was produced by a method which directed the mouse's immune system towards selective recognition of epitopes shared between two broadly divergent serovars. SSMABs was identified by screening in ELISA and Microimmunofluorescence (MIF) assays. Monoclonal antibodies were prepared by immunizing mice with whole C. trachomatis outer membranes (COMC) of the $L_2$ serovar of the B-complex. Mice were boosted with fixed elementary bodies (EBs) of a C-complex isolate, C serovar. The strategy to sensitize B cells to B-complex antigenic determinants, then stimulate proliferation of B cells dedicated to shared determinants by boosting with C-complex, generated four hybrid cell lines, one of which is identified as "SSMAB-1", a secreting species-specific monoclonal antibody.

Example 5: Purification of IgG from Mouse Ascites Fluid

Monoclonal antibody SSMAB-1 was purified by affinity chromatography on a protein A-Sepharose (Bio-Rad Laboratories, Richmond, Cal., U.S.A.) column. SSMAB-1 ascites fluid was centrifuged at 2500 x G for 10 minutes and the supernatant was diluted 10-fold in 0.14 M sodium phosphate buffer, pH 8.0. The diluted ascites was applied to a protein A-Sepharose column that had been equilibrated with the same buffer. The

absorance of the flow-through was determined at 280 nm with a Uvicord (LKB Instruments, Paramus, N.J. U.S.A.) monitor and the column washed until the absorbance of the flow through was 0.01. The bound immunoglobulin was eluted with 0.1 M sodium citrate, pH 4.0, neutralized with HCl and dialyzed at 4°C overnight against 0.1 M sodium borate buffer, pH 8.0. The elution peak fraction was then analyzed for purity by sodium dodecylsulfate polyacrylamide gel electrophoresis (SDS-PAGE, U. K. Laemmli, "Cleavage of Structural Proteins During the Assembly of the Head of Barteviophage T4", Nature (London), 227, 680-685, (1970)) and antigen binding activity by ELISA. This purified IgG was then lyophilized and stored at -20°C until further use.

Example 6: Cyanogen Bromide Cleavage of COMC Proteins

200-500 µg of serotype $L_2$ COMCs were dissolved in 70% formic acid containing a 100-500 molar excess of cyanogen bromide (CNBr) to protein. Digestion was carried out in a sealed tube for 20 hours at room temperature and then evaporated to near dryness at 37°C under a stream of nitrogen. The digest was twice washed with distilled water and dried under nitrogen.

Example 7: Purification of CNBr-Generated Peptides by Reverse-Phase HPLC

The dried CNBr digest was resuspended in 0.05% TFA and was injected onto a Synchropak RP-8 reverse phase HPLC column (4.1 mm x 10 cm). A gradient of 0.05% TFA to 0.05% TFA in 100% acetonitrile was run over a period of 120 minutes at a flow rate of 1 ml/minute. The eluate was monitored at 210 nm with a 0.5 full scale absorbance. The elution profile is shown in Fig. 1A. The entire eluate was collected and a portion of each peak fraction and a sample of the starting material analyzed for reactivity with SSMAB-1 by Western blotting. Western blot analysis of the eluted fractions revealed peak fractions (identified as 1 and 2 on the inset of Fig. 1A) that were immunoreactive and corresponded to immunoreactive material seen in the starting material (shown as the right-most lane of the Western blot insert of Fig. 1A). The position of the immunoreactive fractions in the HPLC elution profile are indicated by the arrows in Fig. 1A. The major immunoreactive fraction (fraction 1) was dried in vacuo, resuspended in 20 mM $NaH_2PO_4$, pH 7 and injected onto the Synchropak RP-8 column previously described. A gradient from 20mM $NaH_2PO_4$ to 20 mM $NaH_2PO_4$ containing 50% acetonitrile was run over 60 minutes at a flow rate of 1 ml/minute. The eluate was monitored at 210 nm with a 0.25 full scale absorbance. The elution profile of this re-chromatographed immunoreactive material is shown in Fig. 1B. All fractions were collected and a portion of each peak fraction analyzed for reactivity with SSMAB-1 by Western blotting (Example 8). The results shown in Fig. 1B, indicated that a single immunoreactive peak (indicated by the arrowhead in the Western blot inset of Fig. 1B) was obtained. The position of this immunoreactive fraction in the HPLC elution profile is indicated by the arrow in Fig. 1B. The reactive mobility of the immunoreactive material shown in the inset of Fig. 1B was calculated to be 6 Kd by comparison to the relative mobility of prestained molecular weight standards (shown flanking the Western blot of Fig. (1B). The remainder of the fraction containing this immunoreactive material was analyzed to determine the sequence of amino acids (Example 9).

Example 8: Identification of Species-Specific Peptides by Western Blotting

A small portion of each HPLC peak fraction was dissolved in sodium dodecylsulfate-polyacrylamide gel (SDS-PAGE) sample buffer. The samples were boiled for five minutes and separated by SDS-PAGE. The peptides were transferred to nitrocellulose paper (NCP, 0.2µM pore size, Schleicher and Schuell, Keene, NH., U.S.A.) in TGM buffer (25mM Tris, 125mM glycine, 20% methanol), at 0.6 Amps current at 4°C for 1.5 hours. The NCP was blocked in PBS containing 3% BSA for 45 minutes and then incubated with SSMAB-1, which had been diluted 1:500 in PBS + 1% BSA, for 1 to 2 hours at room temperature. The NCP was washed three times in PBS, and incubated with peroxidase-conjugated goat anti-(mouse IgG) (heavy and light chain specific; Cappel Laboratories), diluted 1:500 in PBST containing 1% BSA, for one hour at room temperature. Peptides containing a species-specific epitope became visible after incubation of the NCP with peroxidase substrate (60 mg 4-chloro 1-naphthol, 60 µl 30% $H_2O_2$, 20 ml methanol and 100 ml PBS) for 30 minutes at room temperature.

Example 9: Amino Terminal Amino Acid Sequencing of a Species-Specific CNBr Peptide

The immunoreactive fraction shown in Fig. 1B was dried in vacuo, suspended in 0.144 M $NaPO_4$, pH 7.0 and applied to a precycled filter to which 3mg of polybrene and 0.2mg NaCl had been added. The sample was sequenced on an Applied Biosystems (Foster City, Cal., U.S.A.) model 470A Protein Sequencer using standard Edman chemistry and 25% trifluoroacetic acid conversion cycle (cycle 03CPTH). Identification of the PTH-amino acids was performed with an on-line Applied Biosystems model 120A PTH Analyzer using Pen Systems (Cupertino, Cal., U.S.A.) model 2600 Chromatography Software.

The following sequence was obtained, with a 0.87 pmol initial coupling and 95.4% repetitive yield, from this immunoreactive fraction:
X-X-Pro-Tyr-Ile-X-Val-X-X-X-X-Ala-X-Phe-X-Ala. Some residues, indicated by an X, were unidentified due to high background or low yield of that particular amino acid. This partial amino acid sequence was compared to existing $L_2$ MOMP amino acid sequence data (R. S. Stephens, G. Mullenbach, R. Sanchez-Pescador and N. Agabian, "Sequence Analysis of the Major Outer Membrane Protein Gene from Chlamydia trachomatis Serorar $L_2$", J. Bact., 168, 1277-1282, (1986)) to align the species- specific CNBr-derived peptide within $L_2$ MOMP. This comparison indicated that a species-specific epitope resided within the portion of $L_2$ MOMP bounded by Met residues at amino acid positions 262 and 320 of mature $L_2$ MOMP.

Example 10: Synthesis of Peptides

To more precisely localize the species-specific epitope within the immunoreactive CNBr-derived peptide fragment, a series of peptides were synthesized that covered in overlapping fashion the 262 to 320 amino acid residue region of MOMP. Peptides were synthesized on the Applied Biosystems 430A Peptide Synthesizer. The carboxyl terminus amino acid was coupled to the resin by a phenylacetamidomethyl linkage with a substitution of 0.7 mmoles per gram resin. Typically 0.5 mmol of peptide was produced per synthesis. The N-terminal t-BOC was removed with 60% trifluoroacetic acid (TFA) in dichloromethane (DCM) and the alpha-amine neutralized with 10% diisopropylethylamine in dimethylformamide (DMF). t-BOC amino acids (2 mmol) were converted to preformed symmetrical anhydrides by the addition of 1 mmole dicyclohexylcarbodiimide in 2 ml dichloromethane. The side chains of the t-BOC amino acids were protected as follows: Arg(TOS), Asp(OBzl), Cys(4-CH Bzl), Glu(OBzl), His(TOS), Lys(Cl-Z), Ser(Bzl), Thr(Bzl) and Tyr(Br-Z). The t-BOC amino acid Ala, Asn, Gln, Gly, Ile, Leu, Met, Phe, Pro, Trp and Val were not protected. The dicyclohexylamine salt of t-BOC-L-His(TOS) was converted to the free acid by ion-exchange on AG-50-X8(H+) resin (Bio-Rad, Richmond, Cal., U.S.A.) within one hour of coupling. The t-BOC amino acid Asn, Arg, and Gln (2 mmol) were coupled using preformed hydroxybenztriazole (HOBt) active esters formed by the addition of 2 mmol HOBt and 2 mmmol N,N′-dicyclohexylcarbodiimide (DCC). The N-terminal t-BOC was removed from the completed peptide and the peptide resin dried overnight in vacuo.

The peptides were fully deprotected and cleaved from the resin by treatment with anhydrous HF containing 10% anisole for 60 minutes at 0° C. The resin was washed with ethyl acetate and the peptide extracted from the resin with 1.0 N acetic acid. The extract was immediately frozen in liquid $N_2$, lyophilyzed and stored at -20° C until further use.

The crude peptide were resuspended in 0.1% trifluoroacetic acid (TFA) and purified by HPLC on an Altex C-18 (Beckman, Carlsbad, Cal., U.S.A.), 5 micron, 4.1mm x 25cm column. Preparative amounts of peptide (100mg) were purified on a Dynamax (Ralnin Instruments, Woborn, Ma., U.S.A.) C-18 (2.5 x 25cm) column. Solvent A was 0.1% TFA and solvent B was 0.1% TFA, 50% acetonitrile. The 100 minute gradient from solvent A to solvent B was linear. The eluate was monitored at 280 nm and the peak fractions were collected and analyzed by amino acid analysis after 18 hour acid hydrolysis (Millipore/Waters WISP 710B amino acid analyzer). The amino acid sequence of each peptide was confirmed using an Applied Biosystems Model 470A Protein Sequencer and standard Edman chemistry.

A complete list of the peptides synthesized by the this method is shown in Table 1 hereinbelow.

Table 1

MOMP PEPTIDES SYNTHESIZED FOR EPITOPE MAPPING

| Group | PEPTIDE | | INHIBITION WITH ANTIBODY 32.1 |
|---|---|---|---|
| | CNBr MOMP PEPTIDES | | |
| | MOMP 263-279 | FTPYIGVKWSRASFDADC | NO |
| 1 | MOMP 276-292 | FDADTIRIAQPKSATTVC | NO |
| | MOMP 289-308 | ATTVFDVTTLNPTIAGAGDVC | YES |
| | MOMP 305-320 | AGDVKASAEGQLGDTMC | NO |
| | MOMP 306-308 | GDVYC | NO |
| | MOMP 303-308 | AGAGDVYC | NO |
| | MOMP 300-308 | PTIAGAGDVYC | NO |
| 2 | MOMP 297-308 | TLNPTIAGAGDVYC | NO |
| | MOMP 294-308 | DVTTLNPTIAGAGDVYC | NO |
| | MOMP 291-308 | TVFDVTTLNPTIAGAGDVYC | YES |
| | MOMP 289-308 | ATTVFDVTTLNPTIAGAGDVYC | YES |
| | MOMP 295-296 | VTYC | NO |
| | MOMP 294-296 | DVTYC | NO |
| 3 | MOMP 293-296 | FDVTYC | NO |
| | MOMP 292-296 | VFDVTYC | NO |
| | MOMP 291-296 | TVFDVTYC | NO |
| | MOMP 293-299 | FDVTTLNYC | NO |
| 4 | MOMP 292-299 | VFDVTTLNYC | NO |
| | MOMP 291-299 | TVFDVTTLNYC | NO |
| | MOMP 294-302 | DVTTLNPTIYC | NO |
| 5 | MOMP 293-302 | FDVTTLNPTIYC | NO |
| | MOMP 292-302 | VFDVTTLNPTIYC | NO |
| | MOMP 291-302 | TVFDVTTLNPTIYC | YES |
| 6 | MOMP 291-301 | TVFDVTTLNPTYC | NO |
| | MOMP 291-300 | TVFDVTTLNPYC | NO |
| 7 | MOMP 2G11C | TVGDVTTLNPTIYC | NO |
| | MOMP 9G4C | TVFDVTTLNGTIYC | NO |

Example 11: Identification of Synthetic Peptides Containing a Species-Specific Epitope by Competitive Inhibition ELISA

The ability of species-specific monoclonal antibody SSMAB-1 to bind synthetic peptides was determined using a competitive inhibition assay. The peptides used in this study were synthesized with tyrosyl cysteines at their carboxy termini to facilitate both chemical coupling (through the cysteines) and accurate molar quantitation of the peptides in solution (using the molar extinction coefficient of tyrosine at 274.6 nm).

For quantitation, mg amounts of purified synthetic peptide were warmed for 10 minutes at 37° C in 1 ml volumes of PBS-"TWEEN" (0.15 M NaCl, 7 mM $Na_2HPO_4:7H_2O$, 3 mM $NaH_2PO_4:H_2O$, pH 7.4; 0.05% "TWEEN-20"), sonicated for 15 minutes (Branson B32 ultrasonic water bath) and centrifuged for 2 minutes (Beckman Ultrafuge 11). THe optical density of each supernatant was determined at 274.6 nm and the molar concentration of each dissolved peptide was calculated from the molar extinction coefficient of tyrosine at this wavelength. Each peptide solution was diluted in PBS-"TWEEN" to a starting concentration

of 50 µM. Nine four-fold serial dilutions were made in PBS-"TWEEN" to provide a range of peptide concentrations to assay.

A 200 µl aliquot of each peptide dilution in the series was placed in a silanized glass tube. An equal volume of SSMAB-1, in PBS-"TWEEN" containing 1% BSA, at a dilution previously determined to give by itself an ELISA signal at 492 nm of between 1.0 and 2.0, was added. These tubes were shaken (Mini Orbital Shaker, Bellco) at ambient temperature overnight.

Duplicate samples of each peptide-SSMAB-1 mixture were placed at 100 µl/well in ELISA plates previously coated with $L_2$-COMC ELISA antigen. The ELISA was then performed as described in Example 4. If synthetic peptide contained the species-specific epitope, then it would inhibit SSMAB-1 from binding to the COMC adsorbed to the plate. Binding of SSMAB-1 to synthetic peptides was expressed as the percent reduction of absorbance at 492 nm as compared to the control (binding ability of SSMAB-1 to COMC in the absence of synthetic peptide). Peptides that were capable of significantly reducing the ELISA signal in this format are indicated in Table 1.

The inhibition ELISA results indicated that the species-specific epitope recognized by SSMAB-1 consisted of the following sequence, defined by amino acid residues 291 to 302 of mature $L_2$ MOMP: TVFDVTTLNPTI. The outer boundaries of this epitope are clearly defined by the Thr residue (T) at position 291 and the Ile residue (I) at position 302, since omission of either of these residues from this peptide (see Group 5, Table 1 and also Fig. 2) eliminated the ability of peptide to bind SSMAB-1. Two other peptides, containing Gly substitutions at selected residues within the 291-302 region (Group 7, Table 1 and Fig. 3) indicated that replacement of either the Phe residue at position 293 with Gly or replacement of the Pro residue at position 300 with Gly abolish the ability of peptide to bind SSMAB-1. This result indicated that the Phe and Pro residues of this peptide are important recognition elements for the species-specific epitope antibody SSMAB-1.

Example 12: The Use of Synthetic Peptide in the Production of Species-Specific Anti-Chlamydia Antibodies

The synthetic peptide of the sequence TVFDVTTLNPTIYC (Example 11) was used in the production of species-specific anti-*Chlamydia* antibodies. The synthetic peptide was coupled via the carboxy-terminal cysteine to Keyhole Limpet Hemocyanin (KLH, Calbiochem) (La Jolla, Cal., U.S.A.) using the heterobifunctional reagent m-maleimidobenzoyl-N-hydroxysulfosuccinimide ester (s-MBS; Pierce Chemical, Rockford, Il., U.S.A.). KLH (60 mg/ml in 50% mM $NaH_2PO_4$, 10 mM EDTA, pH 7.0 was used to remove the glycerol and other low molecular weight contaminants. The void fraction containing the KLH was concentrated to approximately 50 mg/ml using an Amicon (Danvers, Ma., U.S.A.) positive pressure concentrator. s-MBS was added to the KLH at a molar ratio of 100:1 s-MBS:KLH, assuming a molecular weight of 100,000 daltons for KLH. After a 30 minute reaction at ambient temperature, the free s-MBS was separated from the s-MBS-KLH adduct by gel filtration as described above. The void fraction containing the s-MBS-KLH was added to dry peptide at a molar ratio of 20:1 peptide to KLH. The conjugation reaction was complete after 4 hours at room temperature and the peptide-s-MBS-KLH conjugate stored at -20° C until further use.

The primary immunogen was prepared by emulsifying the peptide-s-MBS-KLH conjugate with an equal volume of Freund's complete adjuvant (Gibco, Grand Island, N.Y., U.S.A.) to yield a final concentration of 2 mg conjugate/ml of emulsion. A female NZW rabbit was pre-bled just prior to initiating the immunization scheme to obtain pre-immune serum. The rabbit was administered a primary intradermal injection of 2 mg of KLH-peptide conjugate prepared as described above, distributed over approximately 20 sites along the back. After four weeks, the rabbit was boosted in the same manner with 2 mg of KLH-peptide conjugate in 50% incomplete Freund's adjuvant. Two weeks later, a final intravenous boost of 400 µg of KLH-peptide conjugate in 1.0 ml PBS (50 mM $NaPO_4$, 10 mM EDTA, pH 7.4) was administered. Ten days later, the rabbit was bled via the ear vein and serum was collected. Mice were immunized each with 50 µg of KLH-peptide in 50 µl of a 50:50 (v:v) mixture of PBS and Complete Freund's Adjuvant via the footpad. One month later, each mouse was boosted with 50 µg of KLH-peptide in 50 µl of a 50:50 (v:v) mixture of PBS:Incomplete Freund's adjuvant administered via intraperitoneal injection. Three days later, immune sera was obtained and the mice used for hybridoma production. The following four techniques were chosen to evaluate the reactivity of these antisera with *Chlamydia* antigen:

(1) analysis of ability to bind COMC ELISA antigen,

(2) ability to Western blot *C. trachomatis* antigens,

(3) ability to bind *C. trachomatis in vivo* and

(4) ability to detect *C. trachomatis* serotypes by microimmunofluorescence (MIF). These four techniques are described in the following four examples.

Example 13: Evaluation of the Titer and Affinity of Anti-Peptide Polyclonal Antibody

Rabbit and mouse antipeptide antisera, prepared according to Example 12, were serially diluted in PBST containing 1% BSA and analyzed for reactivity with serotype $L_2$ COMC by standard ELISA (Example 4). For control purposes, ascites fluid from two species-specific monoclonal antibodies (SSMAB-1 and SSMAB-2) were also diluted and employed in this ELISA on the same plate. The results (Fig. 4) indicated that when immunized with the synthetic peptide, both the rabbit and mouse produced high titers of antibody to the naturally occurring COMC antigen.

Polyclonal anti-peptide antisera from the rabbit was then affinity purified on a synthetic peptide Sepharose column. The peptide was coupled to the Sepharose via a hexanediamine, s-MBS linker. Briefly, 400 ml of a 25% (v:v) slurry of Sepharose CL-4B in 1 M sodium carbonate, pH 11.9 was activated with 5 g of CNBr (in 4 ml acetonitrile). After 2 minutes at 0°C the Sepharose was filtered on a scintered glass funnel, washed with 1.0 liter ice-cold $H_2O$, followed by 1.0 liter phosphate-citrate buffer (PCB; 4 parts 0.2M $NaH_2PO_4$: 1 part 0.1M citric acid) at pH 6.8. The packed resin was resuspended in 100ml PCB containing 20 g hexanediamine and reacted for 2 hours at ambient temperature. The hexanediamine resin was extensively washed with $H_2O$. The resin was resuspended in 50 mM $NaH_2PO_4$, 10 mM EDTA pH 7.0 (P-EDTA) and 2 µmoles of s-MBS were added/mL resin. After 10 minutes the resin was washed with P-EDTA and 2-6 umoles peptide added/mL resin. After coupling overnight at 0°C, the resin was extensively washed with P-EDTA.

The purification of peptide-specific antibody was as follows. One ml of serum was appled to a 2 ml packed column containing the affinity resin described above. The eluate was monitored at 280 nm (2.0 AUFS). The column was washed with 0.2 M Tris-HCl, pH 8.0 until the flow-through was < 0.01 AUFS and the bound immunoglobulin then eluted with 1.0 N acetic acid. The eluate was immediately neutralized with 1.0 M Tris and dialyzed into 0.1 M sodium borate pH 8, 1 mM EDTA containing 0.02% sodium azide. The antibody was stored at 4°C until further use.

This affinity-purified rabbit anti-peptide antibody was quantitated by its absorbance at A280 and assayed for binding to serotype $L_2$ COMC ELISA antigen as described in Example 4. The relative affinity of the purified anti-peptide antibody was shown to be greater than that of purified species-specific monoclonal antibody SSMAB-1, since at an equivalent antibody concentration the affinity-purified rabbit anti-peptide antibody produced an approximately 10-fold greater signal in the ELISA assay (Fig. 5).

Example 14: Western Blotting of $L_2$ EB Antigen Using Mouse and Rabbit Anti-Peptide Polyclonal Sera

1.1 mg of purified serotype $L_2$ EBs (see Example 1) in 500 µl were combined with an equal volume of solublizing buffer (125mM Tris, pH 6.8, 10mM EDTA, 15% SDS, 20% sucrose and 10% mercaptoethanol) and boiled for five minutes before separation on a 10-15% gradient SDS-PAGE gel (see Example 8). Transfer to nitrocellulose (NC) paper was performed in TGM buffer (Example 8) at 60V overnight. The NC paper was blocked for two hours in PBST containing 1% BSA. SSMAB-1, polyclonal immune sera and control sera were incubated with strips of the blocked filter for two hours at the dilutions indicated in Fig. 6.

Filter strips were washed for 3 x 5 minutes in TBST (10mM Tris, pH 8, 150MM NaCl, 0.05% "TWEEN-20"). Anti-(mouse IgG) and anti-(rabbit IgG) ($F_c$ region-specific; Cappel Laboratories, Malvern, Pa., U.S.A.) alkaline phosphatase conjugate were used at a dilution of 1:7500 in TBST for one hour incubation. Washings were as indicated above. Antibodies bound to the filter strips were detected visually after exposure of the strips to alkaline phosphatase substrate, which was comprised of 66ul of nitro blue tetrazolium, (50mg/ml DMF) and 33µl of 5-bromo-4-chloro-3-indolyl phosphate (50mg/ml DMF) in 10 ml of alkaline phosphatase buffer (100mM Tris-HCl, pH 9.5, 100mM NaCl and 5mM $MgCl_2$). The results, shown in Fig. 6, indicated that both the mouse and rabbit anti-peptide antisera reacted specifically with MOMP. Neither pre-immune sera of mouse nor rabbit bound *Chlamydia* antigen in this assay.

Example 15: Detection of Chlamydia In Vivo by Indirect Immunofluorescent Antibody Assay Using Polyclonal Anti-(Species-Specific Peptide) Antibody

HeLa 229 cells were grown as monolayers to confluency on 1 cm diameter circular glass coverslips. These monolayers (approximately $2x10^5$ cells/coverslip) were then infected (Example 1) with $L_2$ EBs. Twenty-four hours after infection, monolayers were rinsed gently with 50 mM sodium phosphate, pH 7.4, 150 mM NaCl (PBS). The rinse was aspirated and the monolayers were fixed with 100% ethanol overnight

at 4° C. Fixed monolayers were hydrated with 1 ml PBS. Each monolayer was overlaid with 35 μl of primary antibody. These primary antibodies consisted of rabbit anti-peptide antiserum and mouse anti-peptide antiserum diluted 1:10 or 1:100 in PBS, rabbit or mouse pre-immune serum diluted 1:10 in PBS, and SSMAB-1 antibody diluted 1:10 or 1:100 in PBS. The monolayers were incubated with primary antibody at 37° C for 30 minutes in a moist environment, rinsed in PBS and then overlaid with 35 μl of either goat anti-(rabbit IgG)-FITC (fluorescein isothiocyanate) or rabbit anti-(mouse IgG)-FITC (heavy and light chain specific; Cappel Laboratories) at a 1:100 dilution in PBS containing 0.02% Evans Blue (Sigma) as a counterstain. The monolayers were incubated with secondary antibody for 30 minutes at 37° C in a moist environment, washed in PBS, then in deionized water and invert-mounted with 10 μl 50mM Tris, pH 8.0 containing 50% (v/v) glycerol on microscope slides. Stained inclusions were visualized at 400x magnification with a Zeiss (Thornwood, N.Y., U.S.A.) fluorescent microscope and photographed on "EKTACHROME 400" (Kodak, Rochester, N.Y., U.S.A.) film.

The results, shown in Fig. 7, indicated that both the rabbit (Fig. 7, panel 1A) and mouse (Fig. 7, panel B) anti-peptide antibodies were able to stain *Chlamydia* inclusions in infected Heha cells. SSMAB-1, the positive control in the experiment also strained these inclusions (Fig. 7, panel C) while all of the control mouse sera (normal rabbit serum, Fig. 7, panel D; normal mouse serum; nm-specific mouse monoclonal) failed to stain inclusions.

## Example 16: Microimmunofluorescence Testing for Anti-Peptide Polyclonal Antibody

To examine the pattern of reactivity of the rabbit anti-peptide antibody with various *C. trachomatis* serotypes, aliquots of this antibody and SSMAB-1 were subjected to microimmunofluorescence testing (S.-P. Wong, C-C. Kuo, J.T. Grayson, "A Simplified Method for Immunological Typing of Trachoma-Inclusion Conjuctivitis-Lymphogranuloma Venereum Organisms", *Infect. Immune*, *7*, 356-360, (1973)). The results, shown in Table 2 hereinbelow indicated that the rabbit anti-peptide antibody reacted with *C. trachomatis* in a species-specific fashion, as did the positive control SSMAB-1, previously determined species-specific reactivity.

Table 2

| MIF testing of rabbit and mouse anti-(M291-302) sera. | | | | | | | | | | | | | | | |
|---|---|---|---|---|---|---|---|---|---|---|---|---|---|---|---|
| C. trachomatis serotype | | | | | | | | | | | | | | | |
| Antibody | A | B | Ba | C | D | E | F | G | H | I | J | K | $L_1$ | $L_2$ | $L_3$ | C.psi + + |
| SSMAB-1 | 512 | 512 | 1024 | 512 | 2048 | 1024 | 32 | 128 | 256 | 256 | 512 | 2048 | 1024 | 1024 | 512 | - |
| Rapeptide | 128 | 128 | 8192 | 128 | 8192 | 8192 | 128 | 8192 | 128 | 238 | 128 | 8192 | 8192 | 8192 | 8192 | - |
| Numbers = the reciprocal of the highest antibody dilution showing a positive reaction with a given serotype<br>- = no reaction | | | | | | | | | | | | | | | | |

It will be appreciated that the instant specification and claims are set forth by way of illustration and not limitation, and that various modifications and changes may be made without departing from the spirit and scope of the present invention.

## Claims

1. An epitope-specific peptide characterizing a common epitope of *Chlamydia trachomatis*, having at least five amino acids, said peptide characterized in that it consists essentially of at least four amino acids having a sequence position of I to XII of the following epitope-specific natural sequence:

```
Thr-Val-Phe-Asp-Val-Thr-Thr-Leu-Asn-Pro-Thr-Ile
 I   II  III IV  V   VI  VII VIII IX  X   XI  XII,
```

wherein said at least four amino acids are in their sequence position, but need not be contiguous to one another, the remaining amino acids being in said natural sequence or being immunogenic equivalents to the amino acids in the natural sequence, said peptide having no more than fifteen consecutive amino acids of natural Chlamydia trachomatis antigen.

2. A peptide according to claim 1, wherein the epitope-specific sequence is
Thr-$AA_1$-Phe-$AA_2$-Pro-$AA_3$-Ile,
wherein $AA_1$, $AA_2$ and $AA_3$ are, respectively, Val, Asp-Val-Thr-Thr-Leu-Asn and Thr, or immunogenic equivalents thereof.

3. A peptide according to claim 1, wherein the epitope-specific sequence is
Thr-Val-Phe-Asp-Val-Thr-Thr-Leu-Asn-Pro-Thr-Ile.

4. An anti-peptide antibody against a common epitope of Chlamydia trachomatis, said antibody produced by immunizing an animal against a peptide of any one of claims 1 to 3.

5. A composition for treating a patient to passively protect the patient against infection by Chlamydia trachomatis comprising and effective amount of an antibody according to claim 4 in a physiologically acceptable diluent.

6. A Chlamydia trachomatis vaccine comprising an effective amount of a peptide according to claims 1 to 3 in a physiologically acceptable diluent.

7. A method for immunopurifying a polyclonal antibody against a common epitope of Chlamydia trachomatis comprising the steps of:

(a) contacting an antiserum preparation raised against a Chlamydia trachomatis antigen with an epitope-specific peptide of any one of claims 1 to 3.

8. A method according to claim 7, wherein said antiserum preparation is fractionated IgG.

9. A method according to claim 7, wherein:

(a) said antiserum preparation is applied to a column containing said peptide fixed to a solid support,

(b) unbound material is eluted, and

(c) epitope-specific antibody is eluted under conditions which disassociate antibody from the solid-phase peptide.

10. A method for assessing the proper performance of an assay method for detecting Chlamydia trachomatis in a sample, the method comprising:

(a) performing a Chlamydia trachomatis assay on the sample by contacting the sample with a first antibody to Chlamydia trachomatis and detecting whether an antigen from the sample becomes bound to said first antibody, and

(b) performing a control reaction by contacting said first antibody, under conditions substantially the same as in step (a), with an epitope-specific peptide of any one of claims 1 to 3 and detecting whether said peptide becomes bound to said first antibody.

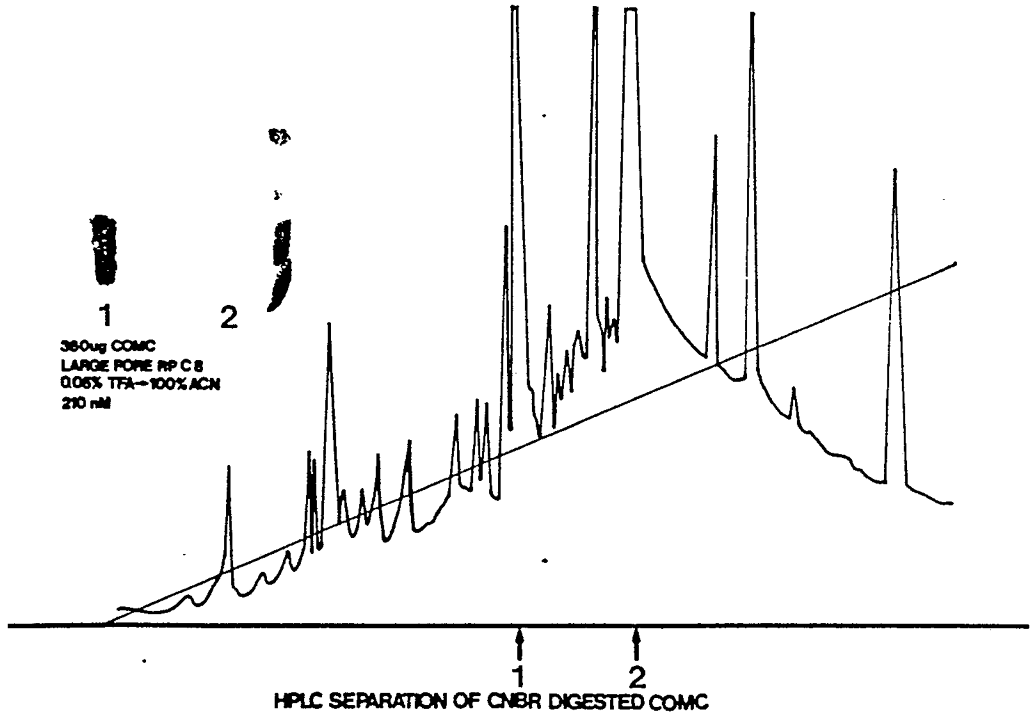


HPLC SEPARATION OF CNBR DIGESTED COMC

FIG.1 A

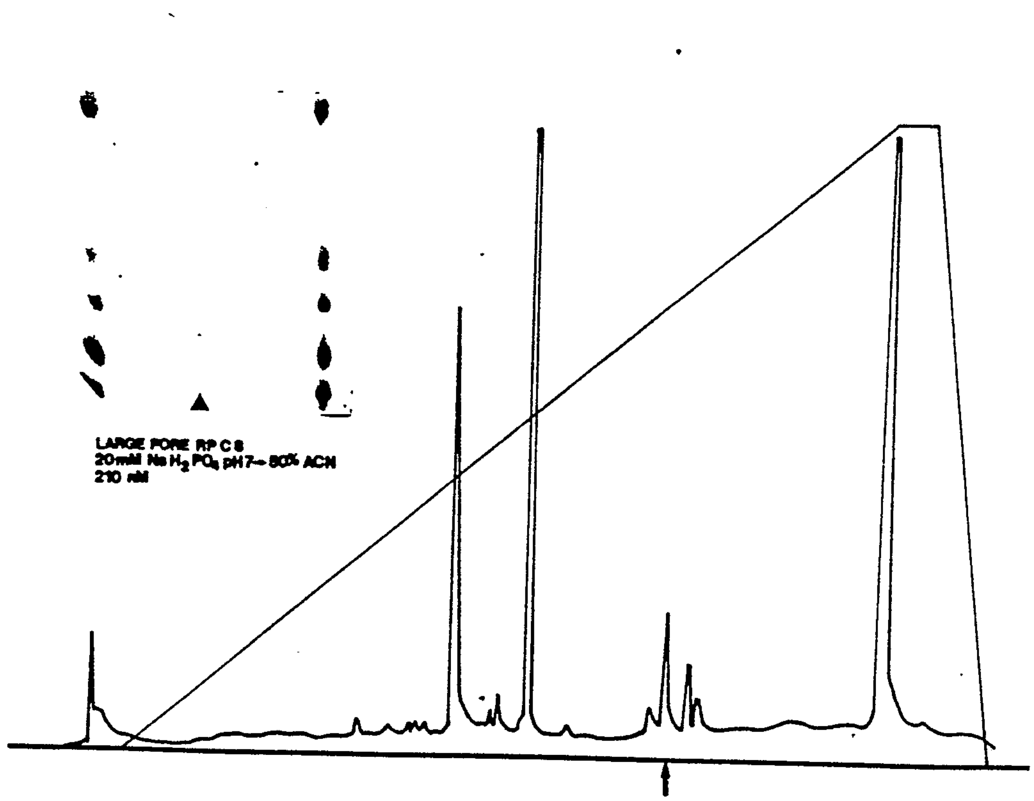


HPLC SEPARATION OF 6Kd CNBR SPECIES SPECIFIC PEPTIDE

FIG.1 B

NH$_2$-TERMINAL BOUNDARY

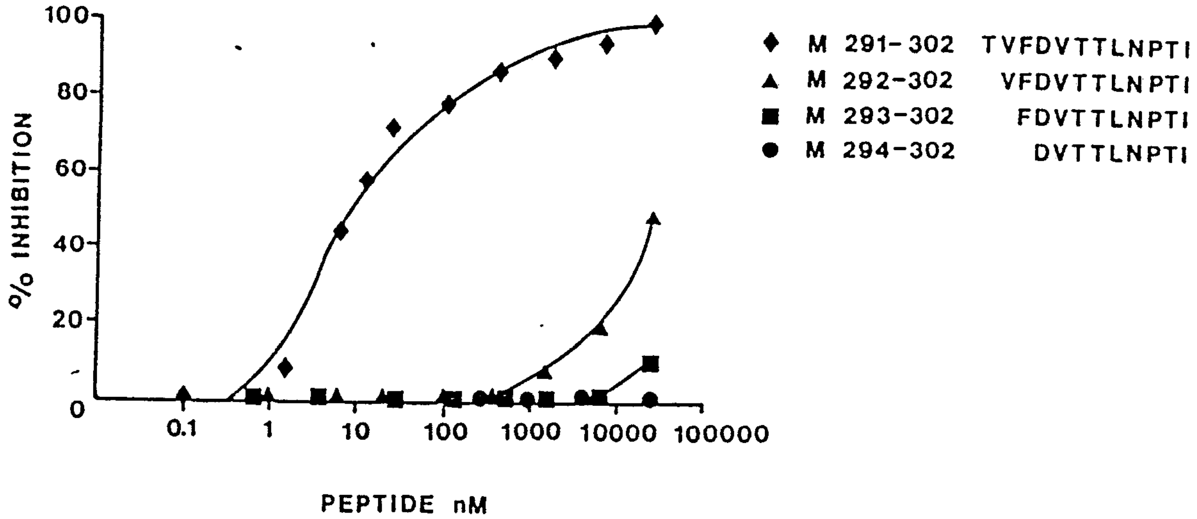


FIG.2A

COOH-TERMINAL BOUNDARY

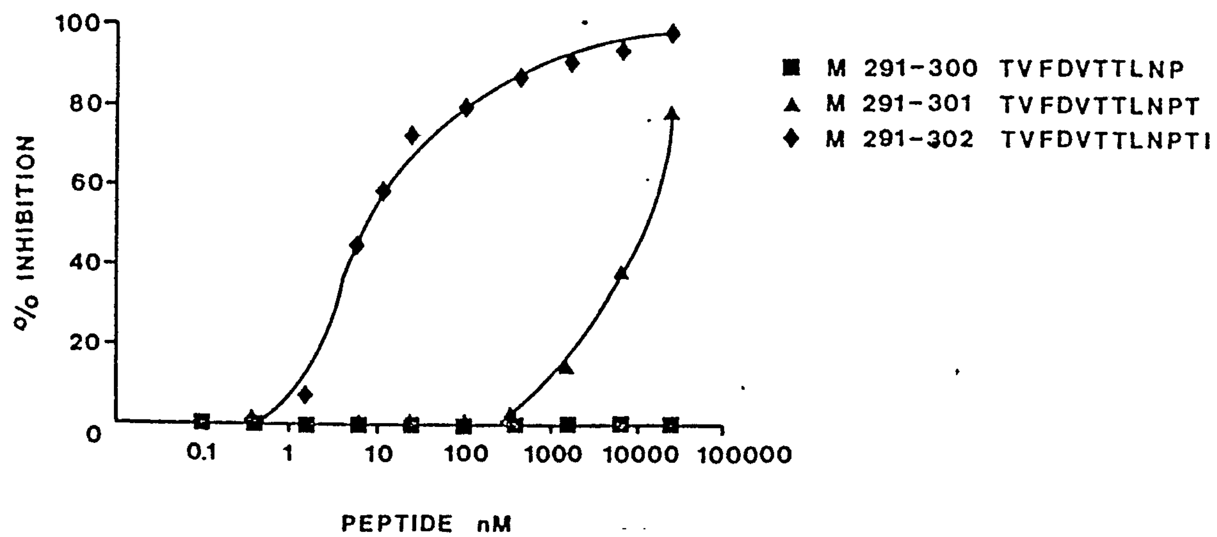


FIG.2B

INTERNAL SUBSTITUTIONS

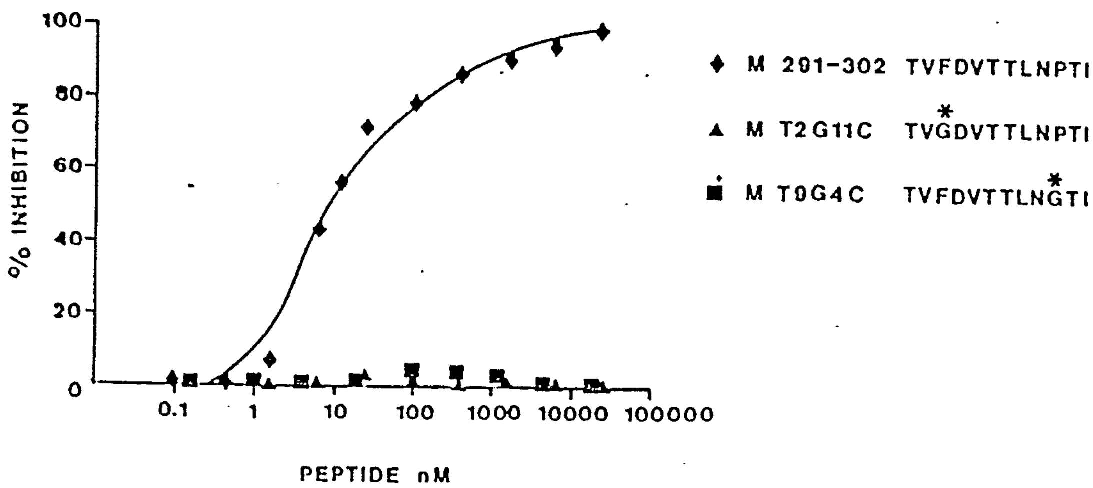


FIG. 3

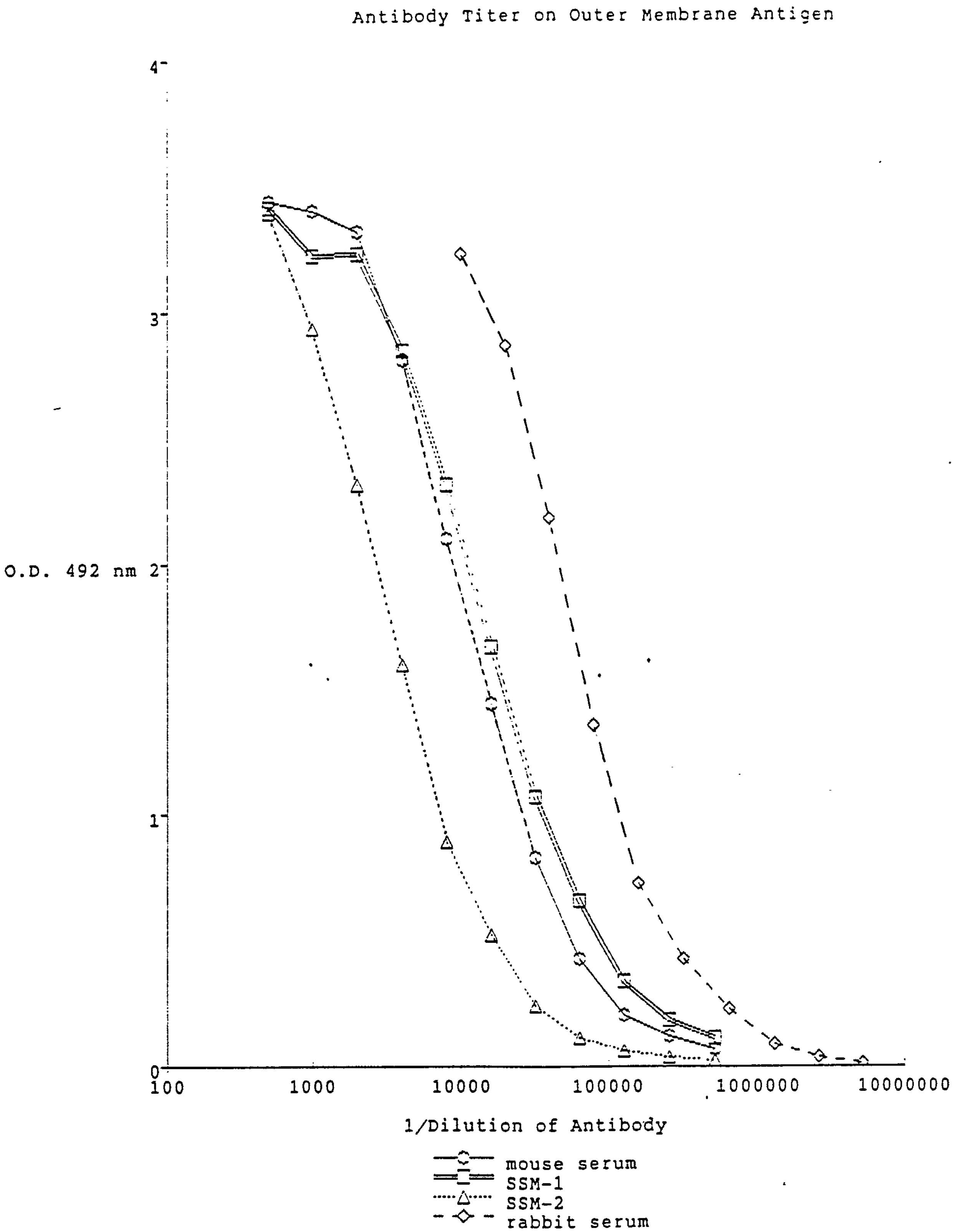

Antibody Titer on Outer Membrane Antigen
O.D. 492 nm
4
3
2
1
0
100
1000
10000
100000
1000000
10000000
1/Dilution of Antibody
mouse serum
SSM-1
SSM-2
rabbit serum


FIG.4

MD248

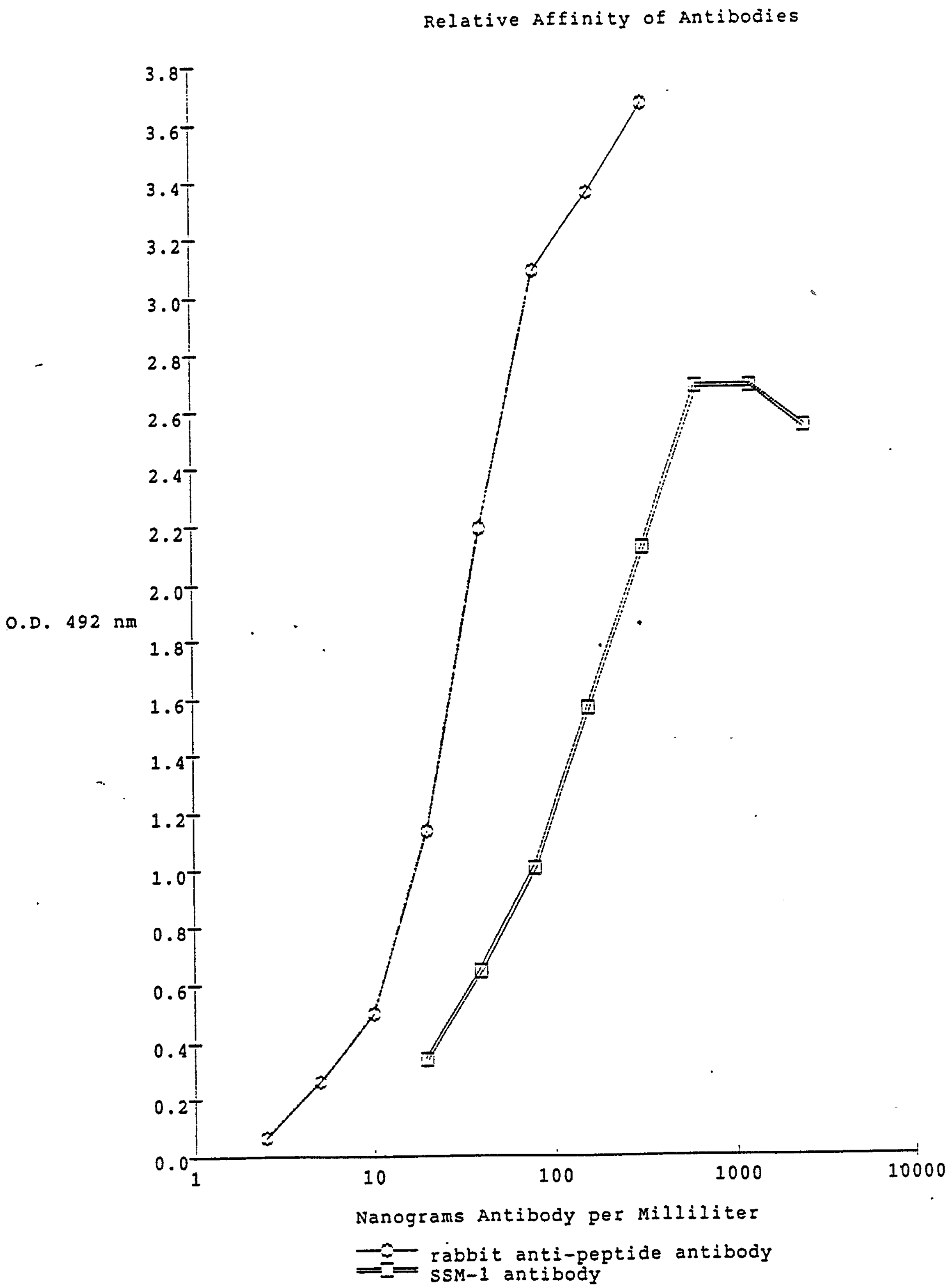
Relative Affinity of Antibodies
O.D. 492 nm
3.8
3.6
3.4
3.2
3.0
2.8
2.6
2.4
2.2
2.0
1.8
1.6
1.4
1.2
1.0
0.8
0.6
0.4
0.2
0.0
1
10
100
1000
10000
Nanograms Antibody per Milliliter
rabbit anti-peptide antibody
SSM-1 antibody

FIG.5

MD248

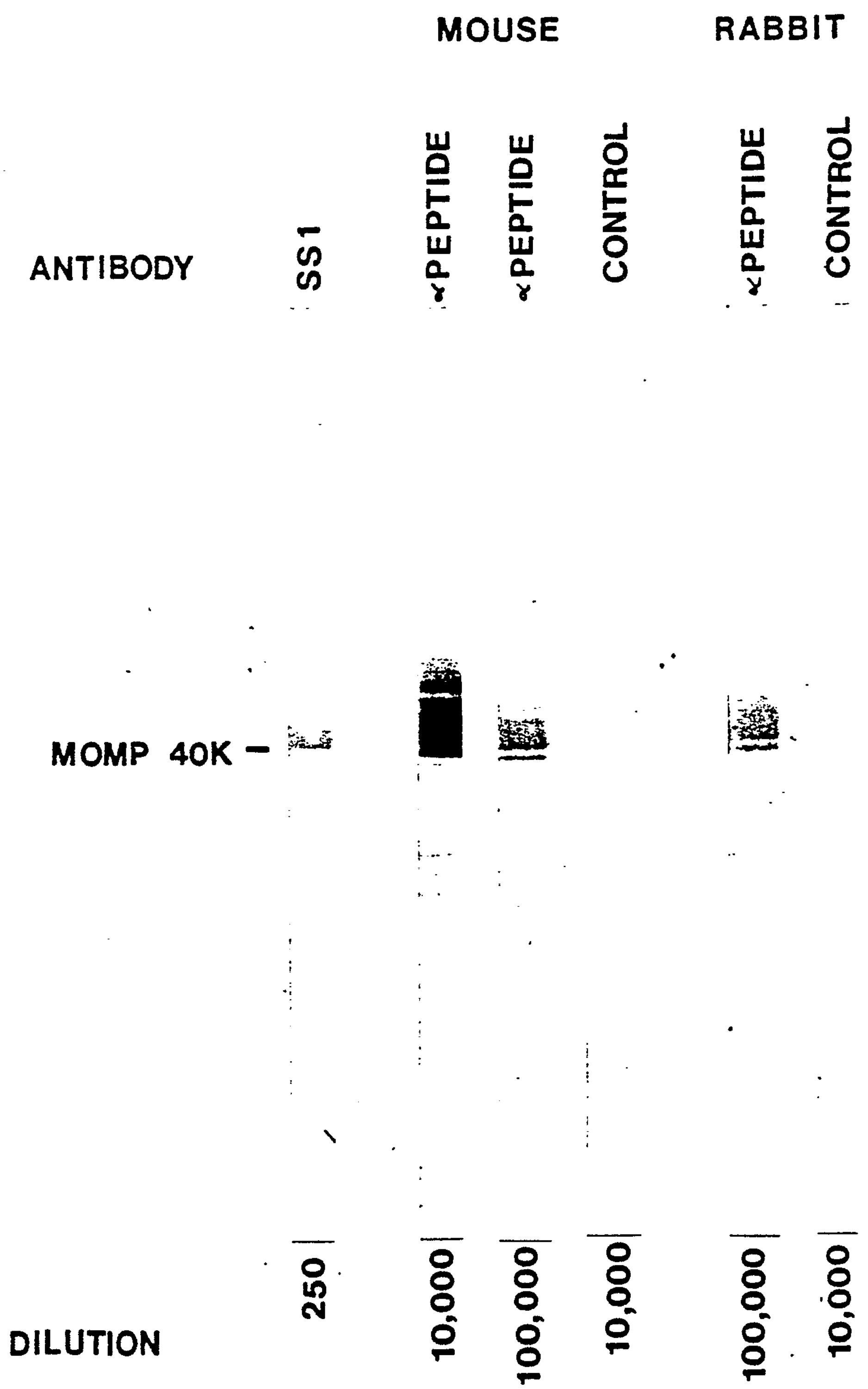
MOUSE
RABBIT
ANTIBODY
SS1
∝PEPTIDE
∝PEPTIDE
CONTROL
∝PEPTIDE
CONTROL
MOMP 40K
DILUTION
250
10,000
100,000
10,000
100,000
10,000

FIG.6

MD248

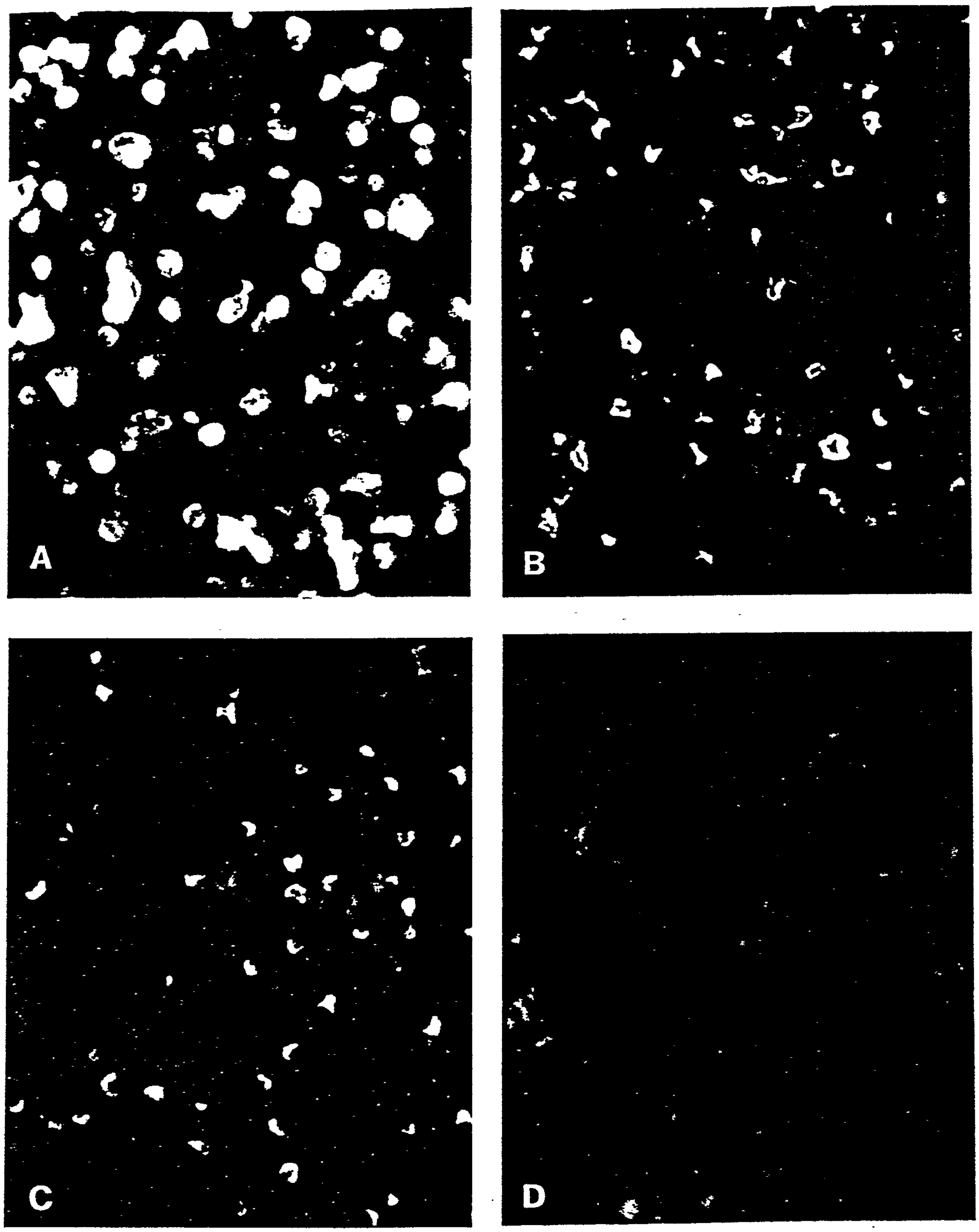


FIG.7